# EUROPEAN PATENT SPECIFICATION

(11) **EP 1 631 202 B1**
(45) Date of publication and mention of the grant of the patent: **07.03.2007**
(21) Application number: 04736659.6
(22) Date of filing: 11.06.2004
(51) Int. Cl.: A61B 17/17

(54) **GUIDE WIRE LOCATION MEANS FOR THE INSERTION OF A PROSTETIC HIP RESURFACING DEVICE**
VORRICHTUNG ZUR POSITIONIERUNG EINES FÜHRUNGSDRAHTES FÜR DIE EINFÜHRUNG EINER HÜFTGELENKKOPFPROTHESE
MOYENS D'EMPLACEMENT DE FIL-GUIDE POUR L'INSERTION D'UN DISPOSITIF DE RESURFACAGE DE HANCHE PROTHETIQUE

(30) Priority: 11.06.2003 GB 0313445
(43) Date of publication of application: 08.03.2006
(73) Proprietor: T J Smith & Nephew Limited, Hull, HU3 2BN (GB)
(72) Inventor: ASHTON, Roger, William, Frank, The Thatch, Stoulton, Worcestershire WR7 4RR (GB); MCMINN, Derek, James, Wallace, 7 Chad Road, Birmingham B15 3EN (GB)
(74) Representative: Connors, Martin L.H.
(86) International application number: PCT/GB2004/002531
(87) International publication number: WO 2004/107993

(56) References cited:
- WO-A-03/055400
- DE-B- 1 164 019
- US-A- 4 896 663
- US-A- 6 156 069
- US-A1- 2002 193 801

## Description

This invention relates to hip resurfacing generally, and in particular to an apparatus for improving the accuracy of installation of a prosthetic hip resurfacing device (femoral component).

In the resurfacing of a patient's hip, installation of the resurfacing device requires that a guide wire for a drill is installed on a chosen axis of the head/neck of the patient's femur. Document US-A-2002/0 193 801, on which the preamble of claim 1 is based, discloses a tool for the location of a guide wire. The chosen axis is identified by the Surgeon through analysis of X-ray or similar technique. Prior to the fitting of the resurfacing device, the femoral head will be machined to a cylindrical shape, and since the axis of this is determined by the drill guide wire, it is important that the guide wire is accurately located, so that the resurfacing device itself can subsequently be accurately fitted.

An object of the invention is to provide an apparatus for improving the accuracy of installation of a prosthetic hip resurfacing device.

According to a first aspect of the invention, guide wire location means for locating, in use, a guide wire at an axis of a neck of a patient's femur comprises a base part for securement to a head of the femur, a part securable to the base part and spherically adjustable relative thereto, a part for directly or indirectly receiving a wire guide, and arranged for planar adjustment, said wire guide receiving part being securable to said spherically adjustable part, and sighting means including a probe having a part engagable with the head and/or neck of the femur.

The sighting means includes a sighting element such as a disc, which is the same size as the interior of a cylindrical saw cutter which will machine the head. By the use of the probe, the surgeon can gauge where the saw cutter will pass when it moves along the axis the guide wire defines, If the saw would cut the femoral neck, and not the head alone, the wire guide receiving part is adjusted accordingly and the sighting repeated until the axis is correct.

There is provided a method of locating a guide wire at an axis of a neck of a patient's femur using guide wire location means of said first aspect of the invention, the method comprising securing said base part to the head of the femur at approximately said axis, appropriately adjusting the attitude of said spherically adjustable part prior to fitting thereto said wire guide receiving part, fitting said wire guide receiving part to said spherically adjustable part, setting its planar position and subsequently adjusting same if necessary in response to engagement of said part of the probe means with the head or neck of the femur, and inserting said guide wire directly or indirectly into the wire guide receiving part upon any adjustment of its planar position having been completed.

The .invention will now be described, by way of example, with reference to the accompanying drawings, in which:
Figure 1 is a sectional side view of guide wire location means of one aspect of the invention in use in a method of accurately locating the guide wire in a chosen axis of a neck of a patient's femur.
Figure 2 is an exploded perspective view of Figure 1, and
Figures 3 and 4 are respectively views corresponding to Figures 1 and 2 for a second embodiment of said guide wire location means.

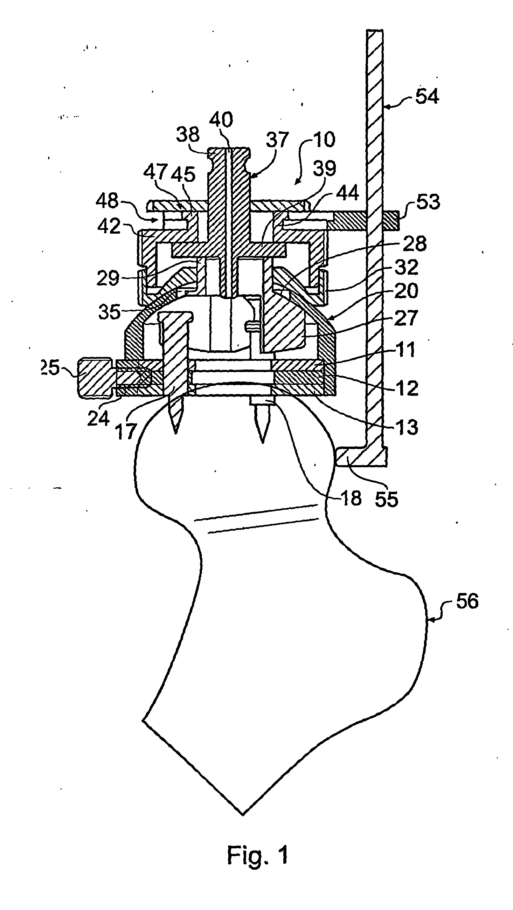

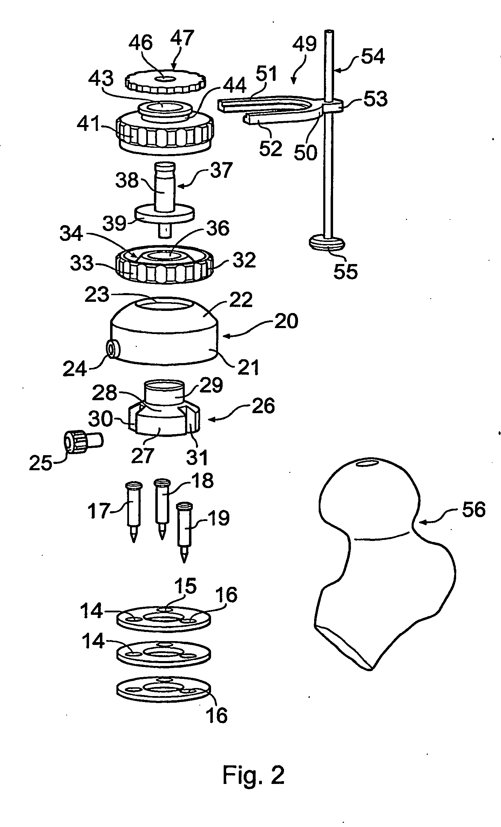

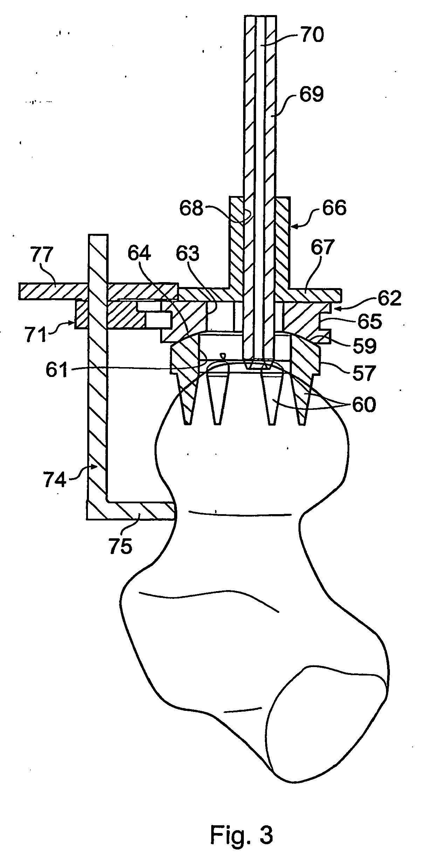

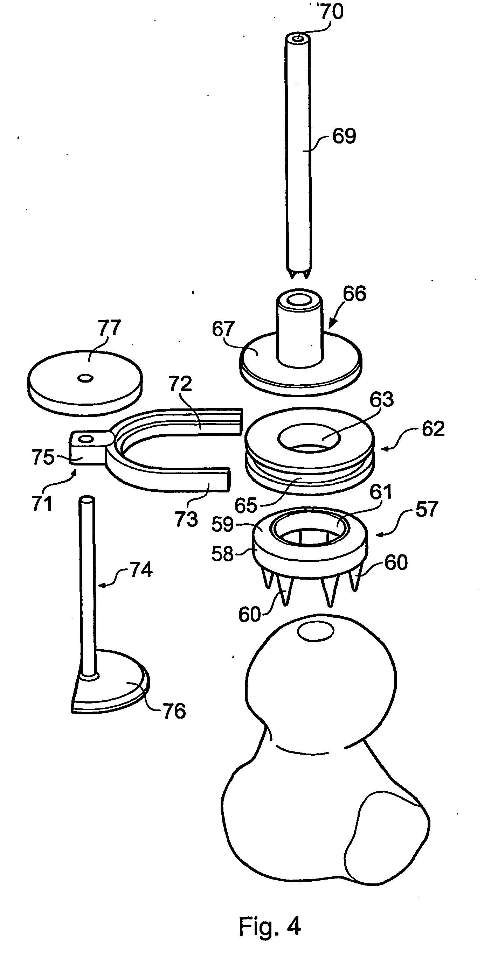

As described in the introduction, it is important with hip resurfacing that the guide wire for the drilling of the head of the femur is accurately located, and the present invention seeks to improve this accuracy as compared to what is presently known. Figures 1 and 2 relate to a first embodiment of guide wire location means of the invention and will be described in relation to the method of operation, whilst Figures 3 and 4 show a second embodiment of the guide wire location means and will be described by way of a second method of operation.

With regard to the first embodiment shown in Figures 1 and 2, the guide wire location means 10 comprises a base part which includes three identical circular plates 11, 12, 13 respectively, each plate having a central circular hole and, in the annular surface therearound, three equi-angularly spaced smaller circular threaded holes 14, 15, 16 respectively. The three plates are placed together with the three larger holes aligned and additionally with each of the smaller holes aligned with one of the smaller holes in the other of the two plates. The base part also includes three headed studs 17, 18, 19 respectively which have pointed ends remote from their respective heads, the respective bodies of the studs extending from the heads having a fine thread therealong. These studs are screwed into the respective aligned holes in the three plates 11, 12, 13 as shown for stud 17 in Figure 1 with its point extending below the lowermost plate 13.

An enclosure 20 has a cylindrical lower portion 21 and a part-spherical upper portion 22, the upper portion having a central circular opening 23 therethrough. The wall thickness of the enclosure 20 is slightly greater with the lower portion 21 than the upper portion 22, but at its open end the interior of the lower portion 21 is outwardly stepped to provide an annular groove so that the assembly of the three plates 11, 12, and 13 can be located inside this open end of the cylindrical lower portion 21, as shown in Figure 1. At the exterior of the position of this grove, there is provided a short, internally threaded boss 24 for receiving a locking screw 25.

Received within the enclosure 20 is an adjustment member 26 which has a generally cylindrical lower part 27 with a part spherical upper surface 28 which substantially matches the interior part-spherical surface of the portion 22 of the enclosure 20. From the centre of this upper surface 28 extends a hollow cylindrical boss 29 which projects through the opening 23 at the top of the enclosure 20 with clearance, in order to allow spherical adjustment motion of the adjustment member 26 within the enclosure 20. Three equi-angularly spaced slots are formed in the lower part 27 of adjustment member 26, these slots extending partly through the upper surface 28. Two of these slots 30, 31 are shown in Figure 2. On the upper part of the part-spherical exterior surface of the enclosure 20 is a circular lock ring 32. This has an exteriorly knurled outer cylindrical part 33, from which extends inwardly a domed portion 34 having a lower, part-spherical undersurface 35 which matches the part-spherical exterior surface at the top of the portion 22 of the enclosure 20. This portion 34 has a central circular hole 36 therein in which the boss 29 is a close fit, with external screw threads on this boss engaging with internal screw threads in the hole 36 so that the lock ring 32 can be screwed to the boss 29, and thus to the adjustment member 26 so as to draw the part-cylindrical surfaces of the member 26 and the lock ring 32 against the cooperating inner and outer part-spherical surfaces respectively of the adjustment member 26 as shown in Figure 1. The three studs 17 to 19 are received respectively in the three slots in the bottom of the adjustment member 26 with clearance, to allow articulation, but preventing rotation while tightening the lock ring 32.

As can be seen from Figure 1, the boss 29 extends through the hole 36 of the portion 34 of the lock ring 32 and has received therein a cannula guide 37 which is the form of an elongated cylindrical body having an external circular flange 39 perpendicular thereto adjacent a reduced diameter end of the body. A fine circular bore 40 extends through the cannula guide 37 for the insertion of the guide wire for a drill (not shown) mentioned above. As shown in Figure 1, the flange 39 rests on the top of the boss 29 with the reduced diameter lower part of the cannula guide being received in the interior of the boss with significant clearance so that the planar position of the cannula guide can be readily adjusted.

The cannula guide can be locked in position by a further circular lock ring 41 which, like the lock ring 32 has a knurled exterior surface. The further lock ring 41 is generally hollow and has its lower end open, with its exterior surface at said lower open end being externally threaded to engage with complimentary internal threads on the part 33 of the lock ring 32. As shown in Figure 1, when the lock ring 32 and the further lock ring 41 are screwed together, the underside of an annular top flange 42 of the further circular lock ring 41 engages against the upper surface of the flange 39 to hold the cannula guide 37 in its adjusted position by virtue of it being forced tightly against the top of the boss 29.

The centre of the flange 42 of the further lock ring 41 is provided with a central circular bore, this extending upwardly through an outwardly lipped boss 44, the annular lip 45 being spaced from, but extending parallel to, the top flange 42 of the further lock ring 41.

Fitted on top of the lip 45 with the body 38 of the cannula guide 37 extending closely through a central circular hole thereof is a sighting disc 47. This thus defines between itself and the upper surface of the flange 42 a stepped annular slot 48. The sighting disc 47 is the same size as the cylindrical saw cutter that will machine the femoral head.

Inserted into this slot is a slider 49 which has an inner portion 50, of the same curvature as the boss 44 and lip 45, and from which extends a pair of spaced parallel arms 51, 52 respectively. The respective interior surfaces of the portion 50 and the arms 51, 52 are configurated to match the shape of the stepped slot, and the spacing of the arms is such that they engage diametrically opposed surfaces respectively of the lipped boss 44 so that the slider can be rotated around the boss. Finally as far as the structure of the guide wire location means is concerned, an extension part 53 of the slider at the opposite side of the portion 50 from that at which the arms extend, is provided with a through hole in which is slidably adjustable rod-like XY probe 54 which with the disc 47 constitutes sighting means of the device. At its lower end the probe is formed with a generally semi-circular contact member 55 arranged to engage the exterior surface of the head of a patient's femur 56. The probe, in conjunction with the sighting disc 47, enable the Surgeon to gauge where the saw cutter will pass when it moves along the axis that the guide wire defines.

The guide wire location means described above is used as follows.

Firstly, the Surgeon rests the base part, comprising the three plates 11 to 13 and the three studs 17 to 19, on the head of the femur as shown in Figure 1, the three plates being engaged together and the three studs each being screwed into one of the three series of three aligned holes in the plates respectively. Initially this positioning of the base part on the head of the femur is such that it lies approximately on the chosen axis of the neck of the femur. A cylindrical drift tool (not shown) is then located into the central aligned larger holes of the plates of the base part and is used to drive the assembly of the plates and the studs into the surface of the bone of the femoral head, thereby securing it in position.

The enclosure 20, adjustment member 26 and lock ring 32 are then fitted as an assembly, with the enclosure being fitted to the base part and secured by the locking screw 25 as shown in Figure 1, the three plates 11 to 13 which are engaged together, being received in the outwardly-stepped groove at the interior of the lower end of the enclosure 20. The lock ring 32, when unscrewed relative to the adjustment member 26, will allow spherical motion of the adjustment member 26 and lock ring 32 relative to the enclosure which is fast with the base part. Thus the Surgeon can choose the correct angular position of the jig, prior to fitting the cannula guide 37.

To optimise the angular position of the adjustment member 26, the surgeon can fit a protractor and probe device (riot shown) into the bore in the boss 29 of the adjustment member and resting on its top surface. This can be used to provide correct angular alignment in both planes using pre-selected features on the femur. When satisfied that the angular alignment is correct, the Surgeon tightens the lock ring 32, with the result that the adjustment member 26 can no longer articulate. It will be noted that the three slots in the adjustment member 26, in which are received the studs 17 to 19 respectively, prevent the adjustment member 26 from rotating about the centre line of the jig during tightening, but still allow articulation of the adjustment member 26 relative to the enclosure.

The Surgeon then fits the cannula guide 37, the further lock ring 41 and the sighting disc 47 as shown in Figure 1. As stated, the sighting disc is the same size as the hollow cylindrical saw cutter and thus represents the final machine diameter of the femoral head prior to the fitting of the resurfacing device. The Surgeon then slides the slider 49 and the XY probe 54 into position so that the contact member 55 touches the high point of the head of the femur, as shown in Figure 1. The Surgeon is thus able visually to judge the position of the sighting disc/cannula guide relative to a sighting mark on the top surface of the slider. This allows the Surgeon then, if necessary, to adjust the cannula guide in a single plane, by slackening the further lock ring 41 and incrementally moving the cannula guide. The slider can then be rotated in its slot 48 in order to make similar adjustments in alternative planes. These alternative planes lie through the axis of the cannula guide, i.e. the Surgeon rotates the probe 54 around the femoral head/neck, testing the XY position of the cannula guide in various vertical planes, 'vertical' meaning along the axis of the cannula guide.

It is important that whilst the femoral head is cut, to a cylindrical shape, the femoral neck/stem below the head is not cut, since this could lead to weakening thereof. Thus having fixed the position of the cannula guide, the Surgeon would probe the head of femur at various heights depending on the shap and degree of malformation - each time comparing the resting position of the probe against the head/neck of the femur with the sighting disc 47 (representing the cylindrical saw cutter that would subsequently be used). In this way he is able to predict where material would be removed and where there would be clearance from the cutter. Thus he can decide if adjustment of the guide 37 is required.

Finally when satisfied with the rotational and planar positioning of the cannula guide 37, the surgeon fixes its position by screwing up the further lock wing 41 so that the arrangement shown in Figure 1 is reached with the cannula guide held in position between the flange 42 and the top of the boss 29. The insertion of the guide wire using the central hole in the cannula guide as a location can then proceed, with this now being set very accurately so that subsequently the drilling of the femur head is similarly of improved accuracy arid is thus at the chosen axis previously identified by the Surgeon through analysis of X-ray or similar technique.

In the second embodiment shown in Figures 3 and 4, the adjustable base part of the first embodiment is replaced with a one-piece base part 57 in the form of an annulus having an exterior cylindrical surface 58 and a part-spherical upper surface 59. Spikes 60 extend from the underside of the surface 58 to be equivalent to the points provided by the studs 17 to 19 of the first embodiment. The annulus defines a central circular opening 61 and the interior surface of this opening can be slightly tapered as shown in Figure 3. This base part 57 will be manufactured in such a manner as to allow the insertion of a Ferro-magnetic core, contained within a Medical Stainless Steel exterior.

Received in engagement with the part 57 on the upper part-spherical surface 59 thereof is an adjustment member 62 which is effectively equivalent to the assembly of the first embodiment formed by the enclosure 20, the adjustment member 26 and the lock ring 32. This adjustment member 62 is basically in the form of a circular annulus having a central circular opening 63 therethrough. The member 62 has its lower surface 64 of part spherical form to match the part-spherical upper surface 59 of the base part 57 to allow spherical adjustment motion as with the first embodiment. The external cylindrical surface of the annular adjustment member 62 is formed with a rectangular annular groove 65. The adjustment member 62 will, like the base part 57, be manufactured in such a manner as to allow the insertion of magnetic material into a Medical Stainless Steel exterior. Accordingly when placed onto the base part 57, the adjustment member 62 will magnetically adhere to the part-spherical surface thereof as shown in Figure 3.

A cannula guide 66 of this second embodiment is of similar form to cannula guide 37 of the first embodiment, but does not have its body continuing below the circular exterior flange which in Figures 3 and 4 is denoted by the numeral 67. Moreover the bore 68 through the guide 66, is no longer fine but is widened to receive an elongated pivot rod 69 therethrough, the rod 69 having a fine bore 70 therethrough for the insertion of the guide wire . The lower end of the pivot rod 69 , which extends below flange 67, is barbed or similarly configured so that it can be pushed into the surface of the bone of the femur head to provide additional support as shown in Figure 3. The cannula guide will be manufactured in such a manner so as to allow the insertion of a Ferro-magnetic core, contained within a Medical Stainless Steel exterior. Thus when placed on the top of the adjustment member 62, the cannula guide will magnetically adhere to the planar surface thereon as shown in Figure 3, with the opening 63 in the member 62 and the opening 61 in the part 57 being greatly oversized relative to the diameter of the pivot rod so as to allow for planar adjustment, as with the first embodiment, of the cannula guide, and thus the pivot rod.

With this second embodiment, as with the first embodiment, a slider 71 of similar form to the slider 49 of the first embodiment, has its arms 72, 73 respectively received in and above the groove 65 formed in the adjustment member 62 so that diametrically opposed inner surfaces of the groove 65 engage respective interior surfaces of a lower part of each of the arms. An XY probe 74 is slidably adjustably received through an opening in an extension part 75 of the slider, this probe 74 having at its lower end a semi-circular contact member 76 to engage the femur as shown in Figure 3. Whilst this embodiment includes, like the first embodiment, a sighting disc 77, this disc is, in this embodiment, received on the end of the circular rod of the probe 74 as shown in Figure 3, so that it lies on the extension part 75 and extends to the top of the adjustment member 62 so that, as shown in Figure 3, it can be in abutting relationship with the outer surface of the flange 67.

In operation, this second embodiment functions in the following manner.

Firstly the one-piece base part 57 is placed on the femoral head so that it lies approximately on the chosen axis. The base part 57 will have at least two or more fixed spikes or studs, with a profile that allows sufficient anchorage into the bone, without having an adverse effect on the fixation of the prosthesis. As with the first embodiment, the application and removal of the base part will be by means of a separate drift tool (not shown). Once the base part has been secured in position, the adjustment member 62 is placed on the top surface thereof, and magnetically adheres thereto as mentioned above. The Surgeon will position the adjustment member 62 to provide appropriate angular position. As with the first embodiment, the spherically adjusted angular position of the adjustment member can be tested with a protractor device (not shown) inserted into the opening 63 of the adjustment member 62. When the Surgeon is satisfied with the angular positioning of the adjustment member 62, the cannula guide 66 will be placed onto the upper surface of the adjustment member 62, as shown in Figure 3, and the cannula guide will thus magnetically adhere to the upper planar surface of the adjustment member 62.

As with the first embodiment, the Surgeon then applies the slider 71 and probe 74 at the groove 65 to determine the optimum position of the cannula guide in one plane. With this embodiment however, the sighting disc 77 is applied to the probe 74 as shown in Figure 3. Alternative sizes of sighting disc 77 can be used depending on the size of prosthesis to be fitted. As shown in Figure 3, the contact member 76 is at a lower part of the femoral head as compared with its position as shown in Figure 1 in the previous embodiment, although this is not a significant difference in that the probe will be used on various points of the head, and/or the neck, of the femur. Again the slider can be rotated in its groove 65 in order to make similar adjustments in alternative planes. Also again, the sighting disc and probe can be used to determine if any incremental adjustment of the guide 66 is required.

When the Surgeon is satisfied with the positioning of the cannula guide, then the pivot rod 69 is inserted into the bore 68 of the cannula guide and pushed down into the surface of the bone to provide additional support as described above. It is then possible to proceed accurately with the insertion of the guide wire through the fine bore 70 in the pivot rod.

Accordingly in both embodiments the accuracy of location of the wire drill guide, and thus ultimately of the drilling, is improved.

Although the example here given used three identical cirucular plates, having three equi-angularly spaced smaller holes to receive three spikes. The skilled person would understand that the invention may have a different number of plates, equi-angularly spaced holes or spikes than three. The invention covers any number of plates and holes and spikes that would equally allow the invention to work.

Likewise threaded holes and screws are described in detail in the main example given to fasten various parts of the guide wire location means together. However other fastening means, for example magnets or clip locks or interference fastening means, may be used that would still enable the invention to work. The skilled person would recognise that the invention covers any fastening means that would allow the invention to work.

## Claims

1. A guide wire location means (10) for locating, in use, a guide wire at an axis of a neck of a patient's femur comprising:
a base part for securement to a head of the femur;
a part securable to the base part,
a part for directly or indirectly receiving a wire guide, and arranged for planar adjustment and sighting means including a probe (54) having a part engagable with the head and/or neck of the femur,
**characterized in that**
the part securable to the base part is spherically adjustable relative thereto; and said wire guide receiving part is securable to said spherically adjustable part.

2. A guide wire location means (10) as claimed in claim 1 in which the sighting means includes a sighting element.

3. A guide wire location means (10) as claimed in claim 2 in which the sighting element is a disc (47).

4. A guide wire location means (10) as claimed in claim 3 in which the disc (47) is the same size as the interior of a cylindrical saw cutter which will machine the head.

5. A guide wire location means (10) as claimed in claim 1 in which the base part includes identical circular plates (11, 12, 13).

6. A guide wire location means (10) as claimed in claim 5 in which the identical circular plates have a central aperture.

7. A guide wire location means (10) as claimed in claim 6 in which the identical circular plates have there around the central aperture in their annular surfaces equi-angularly spaced apertures (14,15,16).

8. A guide wire location means (10) as claimed in claim 7 in which the equi-angularly spaced apertures (14, 15, 16) are three in number.

9. A guide wire location means (10) as claimed in claim 7 or 8 in which the equi-angularly spaced apertures (14, 15, 16) are threaded.

10. A guide wire location means (10) as claimed in anyone of claims 1 to 9 in which the base part includes headed studs (17, 18, 19) which have pointed ends remote from their heads.

11. A guide wire location means (10) as claimed in anyone of claims 5 to 9 in which includes an enclosure (20) having a cylindrical lower portion (21) and a part -spherical upper portion (22), the upper portion having a central opening (23).

12. A guide wire location means (10) as claimed in claim 11 in which the cylindrical lower portion (21) of the enclosure (20) is outwardly stepped to provide an annular groove so that assembly of plates (11, 12, 13) can be located inside this open end of the cylindrical lower portion (21).

13. A guide wire location means (10) as claimed in claim 12 in which an adjustment member (26) is so shaped that it can be received within the enclosure (20).

14. A guide wire location means (10), as claimed in claim 13 in which the adjustment member has a generally cylindrical lower part (27) with a part spherical upper surface (28) which substantially matches the interior part-spherical surface of the portion (22) of the enclosure (20).

15. A guide wire location means (10) as claimed in claim 14 in which the adjustment member (26) has, from the centre of its upper surface (28), a hollow cylindrical boss (29) which projects through the opening (23) at the top of the enclosure (20).

16. A guide wire location means (10) as claimed in any one of claims 14 or 15 in which the lower part (27) of the adjustment member (26) has equi-angularly spaced slots, these slots extending partly through the upper surface (28).

17. A guide wire location means (10) as claimed in claim 16 in which there are three equi-angularly spaced slots.

18. A guide wire location means (10) as claimed in any one of claims 11 to 17 in which a circular lock ring (32) is removably attachable to the upper part of the part-spherical exterior surface of the enclosure (20).

19. A guide wire location means (10) as claimed in any one of claims 15, 16 or 17 in which a cannula guide (37) is so shaped and configured to be received by the boss (29).

20. A guide wire location means (10) as claimed in claim 15 or any one of claims 16 or 17 when dependant on claim 15, in which a cannula guide is so shaped and configured to be received by the boss when the boss (20) extends through a hole (36) of the lock ring (32).

21. A guide wire location means (10) as claim in claim 19 or 20 in which the cannula guide (37) is in the form of an elongated cylindrical body having an external circular flange (39) perpendicular thereto adjacent a reduced diameter end of the body.

22. A guide wire location means (10) as claimed in any one of claims 19, 20 or 21 in which a sighting disc extends closely through a central circular aperture of the body (38) of the cannula guide (37).

23. A guide wire location means (10) as claimed in any one of claims 1 to 4 in which the base part for securement to a head of the femur is a one-piece base part (57).

24. A guide wire location means (10) as claimed in claim 23 in which the one-piece base part (57) is in the form of an annulus having an exterior cylindrical surface (58) and a part-spherical upper surface (59).

25. A guide wire location means (10) as claimed in either claim 23 or 24 in which spikes (60) extend from the underside of the surface (58).

26. A guide wire location means (10) as claimed in any one of claims 1, 2, 3 or 23 to 25 in which a sighting disc (77) extends to the top of an adjustment member.

27. A guide wire location means (10) as claimed in claim 26 in which the sighting disc (77) is received on the end of a circular rod of a probe (74), the probe (74) having at its lower end a contact member (76) to engage the femur.

28. A guide wire location means (10) as claimed in claim 13 to 17 or 26 in which the adjustment member (62) may be manufactured in such a manner as to allow insertion of a magnetic material.

29. A guide wire location means (10) as claimed in any preceding claim in which the base part may be manufactured in such a manner as to allow insertion of a magnetic material.

30. A guide wire location means (10) as claimed in any preceding claim in which comprises magnetic material.

## Patentansprüche

1. Ein Lokalisierungsmittel (10) für einen Führungsdraht zum Lokalisieren, im Gebrauch, eines Führungsdrahts an einer Achse eines Oberschenkelhalses eines Patienten, das Folgendes beinhaltet:
einen Basisteil zur Sicherung an einen Oberschenkelkopf;
einen Teil, der an dem Basisteil gesichert werden kann,
einen Teil zur direkten oder indirekten Aufnahme einer Drahtführung und der zur planaren Einstellung angeordnet ist und ein Sichtmittel, das eine Sonde (54) mit einem Teil, der mit dem Oberschenkelkopf und/oder -hals im Eingriff stehen kann, umfasst, **dadurch gekennzeichnet, dass** der Teil, der an dem Basisteil gesichert werden kann, relativ dazu sphärisch eingestellt werden kann; und wobei der Drahtführungsaufnahmeteil an dem Teil, der sphärisch eingestellt werden kann, gesichert werden kann.

2. Lokalisierungsmittel (10) für einen Führungsdraht gemäß Anspruch 1, bei dem das Sichtmittel einen Sichtbestandteil umfasst.

3. Lokalisierungsmittel (10) für einen Führungsdraht gemäß Anspruch 2, bei dem der Sichtbestandteil eine Scheibe (47) ist.

4. Lokalisierungsmittel (10) für einen Führungsdraht gemäß Anspruch 3, bei dem die Scheibe (47) die gleiche Größe wie das Innere eines zylindrischen Sägemessers, das den Kopf bearbeiten wird, aufweist.

5. Lokalisierungsmittel (10) für einen Führungsdraht gemäß Anspruch 1, bei dem der Basisteil identische, runde Platten (11, 12, 13) umfasst.

6. Lokalisierungsmittel (10) für einen Führungsdraht gemäß Anspruch 5, bei dem die identischen, runden Platten eine zentrale Öffnung aufweisen.

7. Lokalisierungsmittel (10) für einen Führungsdraht gemäß Anspruch 6, bei dem die identischen, runden Platten dort um die zentrale Öffnung herum in ihren ringförmigen Oberflächen gleichwinklig mit Abstand angeordnete Öffnungen (14, 15, 16) aufweisen.

8. Lokalisierungsmittel (10) für einen Führungsdraht gemäß Anspruch 7, bei dem die gleichwinklig mit Abstand angeordneten Öffnungen (14,15,16) drei an der Zahl sind.

9. Lokalisierungsmittel (10) für einen Führungsdraht gemäß Anspruch 7 oder 8, bei dem die gleichwinklig mit Abstand angeordneten Öffnungen (14,15,16) gewindet sind.

10. Lokalisierungsmittel (10) für einen Führungsdraht gemäß einem der Ansprüche 1 bis 9, bei dem der Basisteil Bolzen (17, 18, 19) mit Kopf umfasst, die entfernt von ihren Köpfen zugespitzte Enden aufweisen.

11. Lokalisierungsmittel (10) für einen Führungsdraht gemäß einem der Ansprüche 5 bis 9, das eine Einfassung (20) mit einem zylindrischen unteren Abschnitt (21) und einen teilweise sphärischen oberen Abschnitt (22) aufweist, wobei der obere Abschnitt einen zentralen Durchgang (23) aufweist.

12. Lokalisierungsmittel (10) für einen Führungsdraht gemäß Anspruch 11, bei dem der zylindrische untere Abschnitt (21) der Einfassung (20) nach außen gestuft ist, um eine ringförmige Aussparung bereitzustellen, so dass die Anordnung der Platten (11, 12, 13) in diesem offenen Ende des zylindrischen unteren Abschnitts (21) lokalisiert sein kann.

13. Lokalisierungsmittel (10) für einen Führungsdraht gemäß Anspruch 12, bei dem ein Einstellelement (26) so geformt ist, dass es innerhalb der Einfassung (20) aufgenommen werden kann.

14. Lokalisierungsmittel (10) für einen Führungsdraht gemäß Anspruch 13, bei dem das Einstellelement einen im Allgemeinen zylindrischen unteren Teil (27) mit einer teilweise sphärischen oberen Oberfläche (28) aufweist, die im Wesentlichen der teilweise sphärischen Oberfläche im Inneren des Abschnitts (22) der Einfassung (20) entspricht.

15. Lokalisierungsmittel (10) für einen Führungsdraht gemäß Anspruch 14, bei dem das Einstellelement (26) von dem Zentrum seiner oberen Oberfläche (28) eine hohle, zylindrische Nabe (29), die durch den Durchgang (23) an dem oberen Ende der Einfassung (20) hervorsteht, aufweist.

16. Lokalisierungsmittel (10) für einen Führungsdraht gemäß einem der Ansprüche 14 oder 15, bei dem der untere Teil (27) des Einstellelements (26) gleichwinklig mit Abstand angeordnete Schlitze aufweist, wobei sich diese Schlitze teilweise durch die obere Oberfläche (28) erstrecken.

17. Lokalisierungsmittel (10) für einen Führungsdraht gemäß Anspruch 16, bei dem drei gleichwinklig mit Abstand angeordnete Schlitze vorhanden sind.

18. Lokalisierungsmittel (10) für einen Führungsdraht gemäß einem der Ansprüche 11 bis 17, bei dem ein runder Verschlussring (32) entfernbar an dem oberen Teil der teilweise sphärischen, äußeren Oberfläche der Einfassung (20) befestigt werden kann.

19. Lokalisierungsmittel (10) für einen Führungsdraht gemäß einem der Ansprüche 15, 16 oder 17, bei dem eine Kanülenführung (37) so geformt und konfiguriert ist, dass sie von der Nabe (29) aufgenommen wird.

20. Lokalisierungsmittel (10) für einen Führungsdraht gemäß Anspruch 15 oder einem der Ansprüche 16 oder 17, wenn abhängig von Anspruch 15, bei dem eine Kanülenführung so geformt und konfiguriert ist, dass sie von der Nabe aufgenommen wird, wenn sich die Nabe (29) durch ein Loch (36) des Verschlussrings (32) erstreckt.

21. Lokalisierungsmittel (10) für einen Führungsdraht gemäß Anspruch 19 oder 20, bei dem die Kanülenführung (37) als ein verlängerter, zylindrischer Körper mit einem äußeren, runden, dazu rechtwinkligen Flansch (39), der an ein Ende des Körpers mit verringertem Durchmesser angrenzt, aufweist.

22. Lokalisierungsmittel (10) für einen Führungsdraht gemäß einem der Ansprüche 19, 20 oder 21, bei dem sich eine Sichtscheibe eng durch eine zentrale runde Öffnung des Körpers (38) der Kanülenführung (37) erstreckt.

23. Lokalisierungsmittel (10) für einen Führungsdraht gemäß einem der Ansprüche 1 bis 4, bei dem der Basisteil zur Sicherung an einem Oberschenkelkopf ein einteiliger Basisteil (57) ist.

24. Lokalisierungsmittel (10) für einen Führungsdraht gemäß Anspruch 23, bei dem der einteilige Basisteil (57) als ein Kranz mit einer äußeren, zylindrischen Oberfläche (58) und einer teilweise sphärischen oberen Oberfläche (59) gebildet ist.

25. Lokalisierungsmittel (10) für einen Führungsdraht gemäß Anspruch 23 oder 24, bei dem sich Spitzen (60) von der Unterseite der Oberfläche (58) erstrecken.

26. Lokalisierungsmittel (10) für einen Führungsdraht gemäß einem der Ansprüche 1, 2, 3 oder 23 bis 25, bei dem sich eine Sichtscheibe (77) zu dem oberen Ende eines Einstellelement erstreckt.

27. Lokalisierungsmittel (10) für einen Führungsdraht gemäß Anspruch 26, bei dem dle Sichtscheibe (77) an dem Ende eines runden Stabs einer Sonde (74) aufgenommen wird, wobei die Sonde (74) an ihrem unteren Ende zum Eingriff mit dem Oberschenkelknochen ein Kontaktelement (76) aufweist.

28. Lokalisierungsmittel (10) für einen Führungsdraht gemäß den Ansprüchen 13 bis 17 oder 26, bei dem das Einstellelement (62) in derartiger Weise hergestellt werden kann, um die Einführung eines magnetischen Materials zu erlauben.

29. Lokalisierungsmittel (10) für einen Führungsdraht gemäß einem der vorhergehenden Ansprüche, bei dem der Basisteil in derartiger Weise hergestellt werden kann, um die Einführung eines magnetischen Materials zu erlauben.

30. Lokalisierungsmittel (10) für einen Führungsdraht gemäß einem der vorhergehenden Ansprüche, das ein magnetisches Material beinhaltet.

## Revendications

1. Un moyen de placement de fil-guide (10) destiné à placer, en utilisation, un fil-guide à un axe d'un col du fémur d'un patient comportant :
une partie formant base pour assujettissement à une tête du fémur ;
une partie assujettissable à la partie formant base ;
une partie destinée à recevoir de façon directe ou indirecte un guide-fil, et arrangée pour ajustement plan, et un moyen de visée comprenant une sonde (54) dont une partie peut être mise en prise avec la tête et/ou le col du fémur ; **caractérisée en ce que**
la partie assujettissable à la partie formant base est ajustable de façon sphérique relativement à celle-ci et ladite partie de réception de guide-fil est assujettissable à ladite partie ajustable de façon sphérique.

2. Un moyen de placement de fil-guide (10) tel que revendiqué dans la revendication 1 dans lequel le moyen de visée comprend une unité de visée.

3. Un moyen de placement de fil-guide (10) tel que revendiqué dans la revendication 2 dans lequel l'unité de visée est un disque (47).

4. Un moyen de placement de fil-guide (10) tel que revendiqué dans la revendication 3 dans lequel le disque (47) est de la même taille que l'intérieur d'un dispositif de coupe à la scie cylindrique qui usinera la tête.

5. Un moyen de placement de fil-guide (10) tel que revendiqué dans la revendication 1 dans lequel la partie formant base comprend des plaques circulaires identiques (11, 12, 13).

6. Un moyen de placement de fil-guide (10) tel que revendiqué dans la revendication 5 dans lequel les plaques circulaires identiques ont un orifice central.

7. Un moyen de placement de fil-guide (10) tel que revendiqué dans la revendication 6 dans lequel les plaques circulaires identiques ont autour de l'orifice central dans leurs surfaces annulaires des orifices espacés de façon équiangle (14,15,16).

8. Un moyen de placement de fil-guide (10) tel que revendiqué dans la revendication 7 dans lequel les orifices espacés de façon équiangle (14, 15, 16) sont au nombre de trois.

9. Un moyen de placement de fil-guide (10) tel que revendiqué dans la revendication 7 ou 8 dans lequel les orifices espacés de façon équiangle (14, 15, 16) sont taraudés.

10. Un moyen de placement de fil-guide (10) tel que revendiqué dans n'importe laquelle des revendications 1 à 9 dans lequel la partie formant base comprend des goujons filetés (17, 18, 19) qui ont des extrémités pointues à distance de leurs têtes.

11. Un moyen de placement de fil-guide (10) tel que revendiqué dans n'importe laquelle des revendications 5 à 9, lequel comprend un coffret (20) ayant une portion inférieure cylindrique (21) et une portion supérieure en partie sphérique (22), la portion supérieure ayant une ouverture centrale (23).

12. Un moyen de placement de fil-guide (10) tel que revendiqué dans la revendication 11 dans lequel la portion inférieure cylindrique (21) du coffret (20) est étagée vers l'extérieur afin de fournir une rainure annulaire de sorte que l'assemblage de plaques (11, 12, 13) puisse être placé à l'intérieur de cette extrémité ouverte de la portion inférieure cylindrique (21).

13. Un moyen de placement de fil-guide (10) tel que revendiqué dans la revendication 12, dans lequel un élément d'ajustement (26) est de forme telle à pouvoir être reçu au sein du coffret (20).

14. Un moyen de placement de fil-guide (10) tel que revendiqué dans la revendication 13 dans lequel l'élément d'ajustement a une partie inférieure généralement cylindrique (27) dont une surface supérieure en partie sphérique (28) coïncide substantiellement avec la surface en partie sphérique intérieure de la portion (22) du coffret (20).

15. Un moyen de placement de fil-guide (10) tel que revendiqué dans la revendication 14 dans lequel l'élément d'ajustement (26) a, depuis le centre de sa surface supérieure (28), une protubérance cylindrique creuse (29) qui fait saillie au travers de l'ouverture (23) au sommet du coffret (20).

16. Un moyen de placement de fil-guide (10) tel que revendiqué dans n'importe laquelle des revendications 14 ou 15 dans lequel la partie inférieure (27) de l'élément d'ajustement (26) a des fentes espacées de façon équiangle, ces fentes s'étendant en partie au travers de la surface supérieure (28).

17. Un moyen de placement de fil-guide (10) tel que revendiqué dans la revendication 16 dans lequel il y a trois fentes espacées de façon équiangle.

18. Un moyen de placement de fil-guide (10) tel que revendiqué dans n'importe laquelle des revendications 11 à 17 dans lequel un anneau de verrouillage circulaire (32) peut être attaché de façon démontable à la partie supérieure de la surface extérieure en partie sphérique du coffret (20).

19. Un moyen de placement de fil-guide (10) tel que revendiqué dans n'importe laquelle des revendications 15, 16 ou 17 dans lequel un guide-canule (37) est de forme et de configuration telles à être reçu par la protubérance (29).

20. Un moyen de placement de fil-guide (10) tel que revendiqué dans la revendication 15 ou n'importe laquelle des revendications 16 ou 17 lorsqu'elles dépendent de la revendication 15, dans lequel un guide-canule est de forme et de configuration telles à être reçu par la protubérance lorsque la protubérance (29) s'étend au travers d'un trou (36) de l'anneau de verrouillage (32).

21. Un moyen de placement de fil-guide (10) tel que revendiqué dans la revendication 19 ou 20 dans lequel le guide-canule (37) se présente sous la forme d'un corps cylindrique allongé ayant un flasque circulaire externe (39) perpendiculaire à celui-ci adjacent à une extrémité de diamètre réduit du corps.

22. Un moyen de placement de fil-guide (10) tel que revendiqué dans n'importe laquelle des revendications 19, 20 ou 21 dans lequel un disque de visée s'étend intimement au travers d'un orifice circulaire central du corps (38) du guide-canule (37).

23. Un moyen de placement de fil-guide (10) tel que revendiqué dans n'importe laquelle des revendications 1 à 4 dans lequel la partie formant base pour assujettissement à une tête du fémur est une partie formant base en un morceau (57).

24. Un moyen de placement de fil-guide (10) tel que revendiqué dans la revendication 23 dans lequel la pièce formant base en un morceau (57) se présente sous la forme d'une couronne ayant une surface cylindrique extérieure (58) et une surface supérieure en partie sphérique (59).

25. Un moyen de placement de fil-guide (10) tel que revendiqué dans la revendication soit 23, soit 24 dans lequel des pics (60) s'étendent depuis le dessous de la surface (58).

26. Un moyen de placement de fil-guide (10) tel que revendiqué dans n'importe laquelle des revendications 1, 2, 3 ou 23 à 25 dans lequel un disque de visée (77) s'étend jusqu'au sommet d'un élément d'ajustement.

27. Un moyen de placement de fil-guide (10) tel que revendiqué dans la revendication 26 dans lequel le disque de visée (77) est reçu sur l'extrémité d'une tige circulaire d'une sonde (74), la sonde (74) ayant au niveau de son extrémité inférieure un élément de contact (76) pour se mettre en prise avec le fémur.

28. Un moyen de placement de fil-guide (10) tel que revendiqué dans la revendication 13 à 17 ou 26 dans lequel l'élément d'ajustement (62) peut être fabriqué de telle manière à permettre l'insertion d'un matériau magnétique.

29. Un moyen de placement de fil-guide (10) tel que revendiqué dans n'importe quelle revendication précédente dans lequel la partie formant base peut être fabriquée de telle manière à permettre l'insertion d'un matériau magnétique.

30. Un moyen de placement de fil-guide (10) tel que revendiqué dans n'importe quelle revendication précédente, lequel comporte du matériau magnétique.
